# EUROPÄISCHE PATENTANMELDUNG

(11) **EP 2 366 373 A1**
(43) Veröffentlichungstag der Anmeldung: **21.09.2011**
(21) Anmeldenummer: 11155588.4
(22) Anmeldetag: 23.02.2011
(51) Int. Cl.: A61H 33/10

(54) **Badetank aus einem Acrylatpolymer gefertigt und mit Infrarotstrahlern ausgestattet**

(30) Priorität: 16.03.2010 DE 202010003697 U
(71) Anmelder: Thede, Stefan, 60325 frankfurt am Main (DE)
(72) Erfinder: Thede, Stefan, 60325 frankfurt am Main (DE)
(74) Vertreter: Meyer-Dulheuer, Karl-Hermann

(57) **Zusammenfassung**

Die vorliegenden Erfindung betrifft einen Badetank, welcher mit Wasser befüllbar ist, bestehend aus einem Acrylatpolymer, bei dem es sich um Polyacrylate, Polymethacrylate und/oder deren Copolymeren mit mineralischen Füllstoffen handelt, wobei der Anteil des Acrylatpolymers 30 - 60 Gew.-% beträgt, bevorzugt 40 - 45 Gew.-%, wobei der Füllstoffgehalt im Acrylatpolymer 40 - 70 Gew.-%, bevorzugt 55 ― 60 Gew.-% beträgt und die mineralischen Füllstoffe hydratisiertes Aluminiumoxid, Aluminium Hydroxide und/oder Aluminium Trihydroxide sind.

## Beschreibung

Die Erfindung umschließt einen Badetank, der mit Wasser befüllbar ist. Dieser ist aus einem auf Acryl basierendem Mineralwerkstoff gefertigt und enthält integrierte Infrarotstrahler und unterscheidet sich darin von bereits bekannten Badetanks.

Zu Therapie- und Wellnesszwecken werden Badeeinrichtungen verwendet, die mit Salzwasser oder anderen Flüssigkeiten befüllt werden können. Eine dieser Methoden ist das 'openfloating', bei der Personen im hochkonzentrierten Salzwasser schwerelos an der Oberfläche schweben, ähnlich dem Baden im Toten Meer. Durch unterschiedliche Ausstattungen, wie Farblichtaggregate, sind diese Badeeinrichtungen individuell anwendbar und vielseitig einsetzbar.

In DE 91 05 545 U1 wird der Gebrauch von üblichen Badewannen oder Wasserbecken zu Unterwasser-Heilbehandlungen unter Verwendung von Solen beschrieben. Dabei wird das Salzwasser nach Gebrauch gefiltert und kann somit wieder verwendet werden. Allerdings beinhaltet diese Erfindung weder einen Deckel zum Verschließen des Badetanks noch sind integrierte Zusatzaggregate zu Therapiezwecken enthalten.

Verschließbare Badetanks, wie in DE 198 43 379 A1 beschrieben, finden bei diversen Therapiezwecken wie Schmerz-, Psycho-, Physio-, Sucht-, Antistress- und Sauerstofftherapie Einsatz. Dieser eiförmige Badetank besteht aus zwei Teilen und ist aus glasfaserverstärktem Kunststoff gefertigt. Er kann einzelne Therapieaggregate, wie ein Farblichtaggregat oder eine Kombination aus unterschiedlichen Aggregaten beinhalten. Jedoch ist kein Infrarotstrahler vorhanden. DE 100 63 807 A1 beschreibt einen verschließbaren Badetank, bei dem der Deckel mit einem mit einem Federelement versehbaren Scharnier ausgestattet ist. Dieses ermöglicht einen variablen Einsatz von diversen Entspannungs- und Wellnesseinrichtungen für den Badetank. Auch hier ist kein Infrarotstrahler vorgesehen. Zudem bietet die Verwendung von Glasfaserkunststoffen bei herkömmlichen Badetanks keinen optimalen Schutz vor Ablagerung von Schmutzpartikeln und Mikroorganismen.

Aufgabe dieser Erfindung ist es daher, neue pflegeleichte und hygienische Materialien für Badetanks zu verwenden und somit das Angebot und die Vielseitigkeit von Badeeinrichtungen zu erweitern.

Gelöst wurde die Aufgabe durch die Verwendung eines auf Acryl basierenden Mineralwerkstoffes für den Badetank. Die individuelle Ausstattung des Badetanks wird durch den Einsatz von Infrarotstrahlern erweitert.

Anders als bei bisher verwendeten Badetanks, die hauptsächlich aus Glasfaserkunststoff hergestellt werden, wird ein auf Acryl basierender Mineralwerkstoff verwendet. Als Acrylatpolymer kann Staron (Cheil Industries Inc.) verwendet werden. Bei Staron handelt es sich um ein Polymethylmethacrylat mit mineralischen Füllstoffen, welches zu 40% - 45% aus Acryl-Polymer und zu 55% - 60% aus hydratisierten Aluminiumoxid, Aluminium-Hydroxiden sowie Aluminium-Trihydroxiden besteht.

Staron ist sehr widerstandsfähig. Durch die porenlose Oberfläche können Schmutzpartikel und Mikroorganismen wie Pilze und Bakterien sich nicht festsetzen, wodurch Staron hygienisch und pflegeleicht ist. Gebrauchsspuren lassen sich leicht entfernen. Badetanks aus Staron lassen sich neben dem Privatbereich somit auch im Medizin- und Gesundheitsbereich einsetzen. Zudem ist dieser Werkstoff in einer Vielzahl unterschiedlicher Farben einfärbbar, was die optische Attraktivität steigert.

Bevorzugt ist eine Ausstattung des Badetanks mit Infrarotstrahlern. Diese Infrarotstrahler steigern das Wohlbefinden des Nutzers durch Wärmeentwicklung auf der Haut. Die Wärme dringt in den Körper ein, ohne die Luft im Badetank direkt zu erwärmen. Zudem fördert Infrarotwärme die Giftausschwemmung aus dem Körper.

Der Badetank kann in einer Ausführungsvariante mit einem Deckel vollständig verschließbar sein. Im weiteren kann der Badetank mit Zusatzelementen und Aggregaten wie einer Beschallungsanlage, einer Beleuchtungsanlage, Laserstrahlern, einer Gegensprechanlage, einer Einstiegstufe, einem Heizsystem, einer Bodenheizung, einem Lüftungssystem und/oder einem Sauerstofftherapie-Aggregat ausgestattet werden.

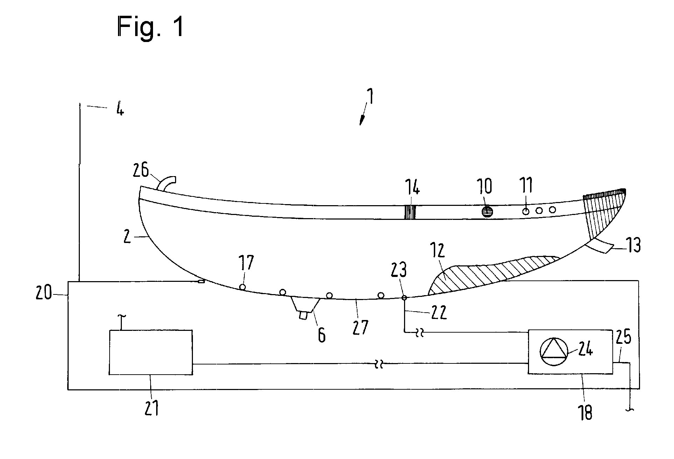

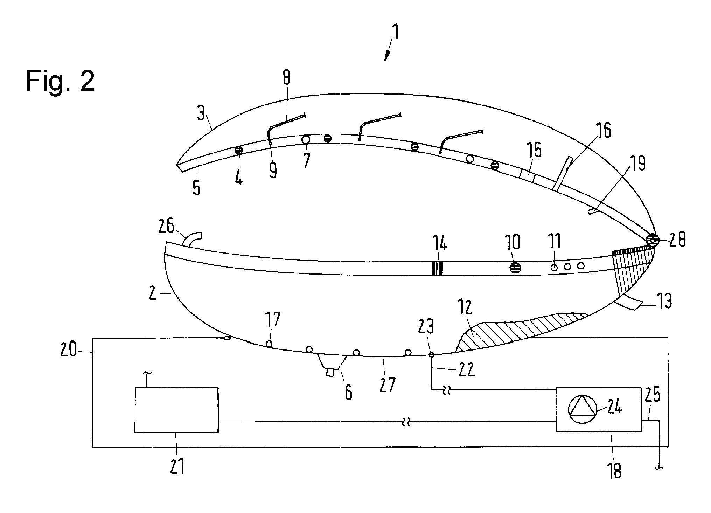

Anhand der Zeichnung 1 (Fig. 1) wird ein Ausführungsbeispiel für einen Badetank, der aus einem Acrylatpolymer gefertigt und mit Infrarotstrahlern ausgestattet ist, ohne Deckelvorrichtung erläutert, in der Zeichnung 2 (Fig. 2) mit Deckelvorrichtung. In der bevorzugten Variante ohne Deckelvorrichtung sind nicht alle Zusatzaggregate zeichnerisch dargestellt.

Die schematische Darstellung Fig. 1 zeigt einen Badetank (1), welcher aus einer Wanne (2) besteht, welche auf einem Sockel (20) steht. Die Infrarotstrahler (4) sind auf dem Wannensockel (20) angebracht und auf die Wanne (2) gerichtet.

Die Wanne (2) ist mit einer oder mehreren Einstiegsstufen (12) ausgestattet. Im Wannenboden (27) ist eine Bodenheizung (17), eine Beschallungsanlage (6), der Wasserein-/ -auslass (23) sowie eine Luftsprudelanlage (13) enthalten. Die Wanne (2) ist zudem mit einer Heizung (14), einer Gegensprechanlage mit Notknopf (10) sowie Bedienelementen (11) für die Gegensprechanlage (10), Infrarotstrahler (4), die Heizung (14) und die Bodenheizung (17) ausgestattet. Die Wanne (2) ist über den Sockel (20) mit einer Wasseraufbereitungsanlage (18) verbunden. Die Wasseraufbereitungsanlage (18) ist mit einer reversierbaren Pumpe (24) versehen, über die das Badewasser über einen Wasserzu-/-ablaufleitung (22) und einen Wasserein-/-auslass (23) in die Wanne (2) eingeleitet werden kann. Zusätzlich ist an der Wanne (2) eine Frischwasserarmatur (26) angebracht.

Das Wasser kann in der Wasseraufbereitungsanlage (18) aufbereitet und in mindestens einem Zwischentank (21) für die nächste Anwendung gespeichert werden. Nicht benötigtes Wasser kann über eine Abwasserleitung (25) abgelassen werden.

Die schematische Darstellung Fig. 2 zeigt einen schließbaren Badetank (1), welcher aus einer Wanne (2) und einem Deckel (3) besteht. Die Wanne und der Wannendeckel sind über ein Scharnier mit einer Schließfeder (28) beweglich miteinander verbunden.

Im Deckelgewölbe (5) sind Infrarotstrahler (4), eine Beleuchtungsanlage (7) und Lichtwellenleiter (8) verlegt, wobei die Endpunkte der Lichtwellenleiter als Lichtpunkte (9) im Deckelgewölbe (5) austreten. Zudem ist eine Lüftungsanlage (15), ein Sauerstofftherapie-Aggregat (16) und Laserstrahler (19) angebracht.

Die Wanne (2) steht auf einem Sockel (20) und ist mit einer oder mehreren Einstiegsstufen (12) ausgestattet. Im Wannenboden (27) ist eine Bodenheizung (17), eine Beschallungsanlage (6), der Wasserein-/ -auslass (23) sowie eine Luftsprudelanlage (13) enthalten. Die Wanne (2) ist zudem mit einer Heizung (14), einer Gegensprechanlage mit Notknopf (10) sowie Bedienelementen (11) für die Beleuchtungsanlage (7), Gegensprechanlage (10), Infrarotstrahler (4), Heizung (14), die Bodenheizung (17) und die Lüftungsanlage (15) ausgestattet. Die Wanne (2) ist über den Sockel (20) mit einer Wasseraufbereitungsanlage (18) verbunden. Die Wasseraufbereitungsanlage (18) ist mit einer reversierbaren Pumpe (24) versehen, über die das Badewasser über einen Wasserzu-/-ablaufleitung (22) und einen Wasserein-/-auslass (23) in die Wanne (2) eingeleitet werden kann. Zusätzlich ist an der Wanne (2) eine Frischwasserarmatur (26) angebracht.

Das Wasser kann in der Wasseraufbereitungsanlage (18) aufbereitet und in mindestens einem Zwischentank (21) für die nächste Anwendung gespeichert werden. Nicht benötigtes Wasser kann über eine Abwasserleitung (25) abgelassen werden.

### Bezugszeichen

- 1: Badetank
- 2: Wanne
- 3: Wannendeckel
- 4: Infrarotstrahler
- 5: Deckenbereich
- 6: Beschallungsanlage
- 7: Beleuchtungsanlage
- 8: Lichtwellenleiter
- 9: Lichtpunkte
- 10: Gegensprechanlage
- 11: Bedienelemente
- 12: Einstiegsstufe
- 13: Luftsprudelanlage
- 14: Heizung
- 15: Lüftungsanlage
- 16: Sauerstofftherapie-Aggregat
- 17: Bodenheizung
- 18: Wasseraufbereitung
- 19: Laserstrahler
- 20: Wannensockel
- 21: Zwischentank
- 22: Wasserzu-/-ableitung
- 23: Wasserein-/-auslass
- 24: Pumpe
- 25: Abwasser
- 26: Frischwasserarmatur
- 27: Wannenboden
- 28: Scharnier mit Schließfeder

## Patentansprüche

1. Badetank, mit Wasser befüllbar und einem Deckel verschließbar, **bestehend aus** einem Acrylatpolymer, bei dem es sich um Polyacrylate, Polymethacrylate und/oder deren Copolymeren mit mineralischen Füllstoffen handelt, wobei der Anteil des Acrylatpolymers 30 - 60 Gew.-% beträgt, bevorzugt 40 - 45 Gew.-%, wobei der Füllstoffgehalt im Acrylatpolymer 40 - 70 Gew.-%, bevorzugt 55 ― 60 Gew.-% beträgt und die mineralischen Füllstoffe hydratisiertes Aluminiumoxid, Aluminium-Hydroxide und/oder Aluminium-Trihydroxide sind.

2. Vorrichtung gemäß Anspruch 1, **dadurch gekennzeichnet, dass** das Acrylatpolymer Polymethylmethacrylat ist.

3. Vorrichtung gemäß Anspruch 1-2, **dadurch gekennzeichnet, dass** Infrarotstrahler integriert sind.

4. Vorrichtung gemäß Anspruch 1-3, **dadurch gekennzeichnet, dass** der Badetank über eine Wasseraufbereitungsanlage mit einer Wasserfüllung befüllbar ist.

5. Vorrichtung gemäß Anspruch 1-5, **dadurch gekennzeichnet, dass** die Wasserfüllung in einem Zwischentank bevorratet ist.

6. Vorrichtung gemäß Anspruch 1-5, **dadurch gekennzeichnet, dass** der Badetank mit einer Beschallungsanlage ausgestattet ist.

7. Vorrichtung gemäß Anspruch 1-6, **dadurch gekennzeichnet, dass** der Badetank mit einer Beleuchtungsanlage ausgestattet werden kann, welche unterschiedliche Lichtfarben beinhaltet.

8. Vorrichtung gemäß Anspruch 1-7, **dadurch gekennzeichnet, dass** in dem Deckel des Badetanks Lichtwellenleiter verlegt werden können, deren offene Enden als Lichtpunkte in den der Wanne zugewandten Deckelgewölben enden.

9. Vorrichtung gemäß Anspruch 1-8, **dadurch gekennzeichnet, dass** der Badetank mit einem dem Deckengewölbe zugewandten Laserstrahler versehen ist.

10. Vorrichtung gemäß Anspruch 1-9, **dadurch gekennzeichnet, dass** der Badetank mit einer Gegensprechanlage ausgestattet ist.

11. Vorrichtung gemäß Anspruch 1-10, **dadurch gekennzeichnet, dass** der Badetank mit einer Vielzahl von Bedienelementen ausgestattet ist.

12. Vorrichtung gemäß Anspruch 1-11, **dadurch gekennzeichnet, dass** der Badetank mit mindestens einer Einstiegsstufe und einer Kopfstütze ausgestattet ist.

13. Vorrichtung gemäß Anspruch 1-12, **dadurch gekennzeichnet, dass** der Badetank beheizbar ist und mit einem Lüftungssystem ausgestattet ist.

14. Vorrichtung gemäß Anspruch 1-13, **dadurch gekennzeichnet, dass** der Badetank mit einem Sauerstofftherapie-Aggregat ausgestattet ist.

15. Vorrichtung gemäß Anspruch 1-14, **dadurch gekennzeichnet, dass** der Badetank mit einem Massage-Aggregat ausgestattet ist.
